# FASCICULE DE BREVET EUROPEEN

(11) **EP 2 411 212 B1**
(45) Date de publication et mention de la délivrance du brevet: **24.06.2020**
(21) Numéro de dépôt: 10710869.8
(22) Date de dépôt: 05.03.2010
(51) Int. Cl.: B32B 27/12

(54) **PARTIE À CROCHETS D'UNE FERMETURE AUTO AGRIPPANTE À CROCHETS ET BOUCLES COMPRENANT UN ÉLÉMENT À CROCHETS FIXÉ À UN STRATIFIE ELASTIQUE**
NACHGIEBIGES LAMINAT UND VERSTÄRKTES VLIES
RESILIENT LAMINATE AND REINFORCED NONWOVEN FABRIC

(30) Priorité: 23.03.2009 FR 0901339
(43) Date de publication de la demande: 01.02.2012
(60) Demande divisionnaire: 20170217.2
(73) Titulaire: Aplix, 44850 Le Cellier (FR)
(72) Inventeur: MARCHE, Thierry, F-44450 La Chapelle Basse Mer (FR); MOINARD, Nathalie, F-44300 Nantes (FR)
(74) Mandataire: Eidelsberg, Olivier Nathan
(86) Numéro de dépôt international: PCT/FR2010/000190
(87) Numéro de publication internationale: WO 2010/109087

(56) Documents cités:
- WO-A1-01/53076
- WO-A1-2009/042556
- US-A1- 2003 109 843
- US-A1- 2005 060 849
- US-B1- 6 195 850
- US-B1- 6 245 401

## Description

La présente invention se rapporte à un stratifié comportant au moins un film élastique fixé par de l'adhésif à au moins une couche de non tissé, notamment deux couches de non-tissé respectivement supérieure et inférieure. Ce genre de stratifié est utilisé, en particulier, dans des applications dans le domaine des vêtements, notamment jetables, tels que des couches-culottes et des dispositifs contre l'incontinence pour adulte, ou dans le domaine médical, sous la forme de bandage élastique. Dans les couches-culottes, ces stratifiés sont classiquement utilisés dans les parties formant la ceinture autour de la taille du bébé. Dans le cas d'une application particulièrement avantageuse pour les couches-culottes, ces stratifiés sont utilisés pour réaliser les rabats élastiques qui garantissent que la couche-culotte est maintenue fermement sur le bébé, notamment élastiquement, le rabat, formant une partie à crochet, portant par exemple des éléments mâles d'un auto agrippant, notamment des crochets, destinés par exemple à coopérer avec des boucles d'une partie centrale de la ceinture de la couche culotte.

On connaît déjà, dans l'art antérieur, des stratifiés de ce genre, notamment de la demande de brevet français n° 05 11343 déposée le 8 novembre 2005 au nom de la demanderesse. Les stratifiés qui y sont décrits sont destinés notamment à être fixés d'un côté au châssis de la couche et de l'autre côté à une bande étroite comportant des crochets. Les non-tissés constituant ces stratifiés de l'art antérieur sont soit des non tissés qui n'ont pas de capacité d'extension, soit des non tissés qui peuvent être étirés dans une direction. Dans le cas des non tissés non extensibles, il est nécessaire, pour que le stratifié ait des capacités élastiques, de l'«activer», notamment en cassant partiellement les fibres le constituant ou du moins en en diminuant la cohésion, notamment par attaque avec des lames, pour ainsi conférer une élasticité au stratifié dans son ensemble.

De US-A-6245401, on connaît un stratifié, comportant une strate, constituée d'un film élastique et de deux films moins élastiques mais perméables à la vapeur d'eau, et une couche de non tissé, qui peut être utilisé en tant que le recouvrement extérieur imperméable aux liquides d'un châssis de couche culotte.

De US-A-5773374, on connaît un stratifié comportant deux couches de non tissé qui prennent en sandwich un élément élastique, cet élément élastique étant constitué d'une âme en un matériau élastique et de deux peaux de part et d'autre de l'âme qui ont été préalablement étirées et solidarisées à l'état étirées à l'âme, elle même à l'état étiré, de sorte que une fois solidarisées les peaux se rétractent en même temps que l'âme pour former des ondulations. L'élément constitué de l'âme et des deux peaux est ainsi doué d'élasticité entre sa longueur au repos et sa longueur maximale lorsqu'il est étiré de sorte que les peaux ne forment plus d'ondulations. Le procédé de fabrication du stratifié est ainsi complexe et prend beaucoup de temps.

De WO-A-01/53076, on connaît un stratifié comportant deux couches de non tissés prenant en sandwich une strate d'une seule pièce en un matériau élastique. En outre, entre la strate élastique et une couche de non tissé, il est interposé un film non élastique de renfort , avec interposition de colle des deux côtés du film de renfort, pour le coller d'une part au non tissé et d'autre part à la strate élastique. Cet assemblage par collage de plusieurs films fait que le procédé de fabrication du stratifié est complexe et prend beaucoup de temps.

De US 2005/0060849A1, il est connu de co-extruder deux bandes, l'une en un matériau élastomérique et l'autre en un matériau thermoplastique dans lequel on peut mouler des crochets, la co extrusion se faisant sur une couche support, sans interposition de colle, puis on sépare, en tirant dans la direction CD, les deux bandes co extrudées l'une à l'autre pour obtenir au final des éléments à crochets individuels.

US6195850 B1 décrit des systèmes de fermeture à crochets et boucles pour des couches-culottes.

US5773374 A décrit un matériau élastique destiné à être utilisé dans les vêtements, par exemple dans les couches-culottes.

Dans un cas comme dans l'autre, il est difficile soit de réaliser le stratifié, notamment parce qu'il est constitué d'un grand nombre de pièces, ce qui le rend complexe, soit d'assurer à grande vitesse la fixation du stratifié soit au châssis de la couche-culotte soit au rabat à crochets sauf à utiliser des non tissés supérieur et inférieur ayant des grammages élevés, ce qui a un effet défavorable sur les coûts de production à grande échelle.

Suivant un autre art antérieur, il est connu de traiter des zones du ou des non tissés pour annihiler les propriétés élastiques du stratifié au niveau de ces zones, par exemple par adjonction d'une couche complémentaire de film rigide ou de non tissé en sur épaisseur du stratifié. Cependant, dans ce cas également, le traitement est coûteux et peu propice à une mise en oeuvre sur des lignes de fabrication rapides et à grande échelle.

La présente invention vise à surmonter les inconvénients de l'art antérieur en proposant un stratifié, comportant au moins un film élastique fixé par adhésif entre deux couches de non-tissé, que l'on peut obtenir d'une manière très simple, particulièrement adaptée à une fabrication à très grande échelle et qui dans le même temps est plus simple à assembler à une couche-culotte, et notamment au châssis de la couche, et auquel il est plus facile, également, d'assembler un élément de largeur étroite comportant des crochets pour former une partie à crochets pour permettre la fermeture de cette couche-culotte.

Suivant l'invention, une partie à crochets d'une fermeture auto agrippante à crochets et boucles, notamment pour une couche culotte, est telle que définie à la revendication 1, des perfectionnements étant définis aux sous revendications 2 à 9.

Suivant l'invention, on entend d'une seule pièce, une strate qui n'est pas constituée par la fixation ensemble de plusieurs pièces, notamment films, à l'aide de moyen de fixation intermédiaire, telle qu'un adhésif ou de la colle. En particulier, la strate d'une seule pièce est une strate obtenue en extrudant un matériau ou en co-extrudant plusieurs matériaux.

En particulier, ladite une zone élastique en forme de film est en contact direct avec au moins une couche de non tissé, avec interposition d'une couche d'adhésif, notamment de colle.

De préférence, le stratifié a une largeur mesurée dans la direction transversale entre les deux bords gauche et droit d'extrémité, une épaisseur mesurée dans la direction d'empilement des strates du stratifié et une longueur dans la direction perpendiculaire à la direction en largeur et la direction en épaisseur, les couches ou nappes de non-tissé et d'adhésif s'étendant sur toute la largeur du stratifié, tandis que la somme des largeurs de la ou des zone(s) en forme de film(s) élastique(s) est strictement inférieure à la largeur du stratifié et la somme des largeurs de la ou des zones en forme de film(s) de renforcement est également strictement inférieure à la largeur du stratifié.

En particulier, suivant un mode de réalisation préféré de l'invention, la ou les zones en forme de film(s) de renforcement et la ou les zone(s) en forme de film(s) élastique(s), avant leur fixation à la au moins une couche de non tissé, sont formés en une pièce ou film unique par coextrusion de sorte que leurs bords latéraux viennent en contact mutuel. Les matières des films de renforcement et du ou des film(s) élastique(s), par exemple des matières thermoplastiques et élastométiques, sont extrudées contiguës les unes aux autres, de sorte que les films de renforcement et le film élastique forment une pièce unique.

De préférence, la ou les zones en forme de films de renforcement sont constituées d'une matière, issue d'une formulation ou recette intégrant notamment des matières thermoplastiques, éventuellement élastomériques, qui confère à iso charge suivant la méthode de test d'élongation décrite ci après des propriétés moindres d'élongation que la ou les zones en forme de films élastiques. De préférence, ces zones sont inélastiques.

Avec la partie à crochets suivant l'invention, on peut maintenant obtenir, à grammage égal des non tissés utilisés, une résistance améliorée à la traction latérale et une bonne fixation de la partie à crochets au niveau de ses bords au châssis d'une couche culotte, ou, pour un grammage plus faible de l'un au moins des non tissés utilisés, notamment compris entre 5 et 20 g/m², de préférence entre 5 et 15 g/m², en particulier entre 5 et 10 g/m², une aussi bonne fixation que pour des parties à crochets de l'art antérieur comportant des non tissés de plus fort grammage.

Suivant un mode de réalisation préféré, la nappe à crochets, constituée d'une bande de base et d'une pluralité d'éléments d'accrochage issus de la bande, est fixée, par exemple par collage, calandrage à chaud, soudure à ultrasons ou analogue, sur une face extérieure du au moins un non tissé formant le stratifié, au niveau du bord sur une zone qui se trouve au dessus ou en dessous d'une des zones de renforcement, notamment latérales.

Suivant un autre mode de réalisation, la nappe à crochets, constituée d'une bande de base et d'une pluralité d'éléments d'accrochage issus de là bande, est fixée, par exemple par collage, calandrage à chaud, soudure à ultrasons ou analogue, sur une face extérieure d'au moins une des zones de renforcement latérales et/ou central.

Suivant un mode de réalisation préféré de l'invention, les deux couches d'adhésif, notamment de colle, entre respectivement chaque non-tissé et la strate d'une pièce ou d'un seul tenant constituée d'au moins deux zones de renforcement en forme de film et d'au moins une zone élastique en forme de film sont réalisées de telle manière qu'entre le non-tissé et la ou les partie(s) formant une zone élastique de la strate intermédiaire, l'adhésif se trouve déposé sous forme de bandes séparées les unes des autres par des bandes sans adhésif.

Soit le non-tissé a une capacité d'extension dans la direction transversale, auquel cas les bandes de colle séparées permettent d'assurer au stratifié une élasticité, soit le non-tissé choisi n'est pas extensible, et il est alors activé en étirant l'ensemble transversalement de sorte que sur les bandes de colle vont s'agglutiner des amas de fibres de non-tissé, tandis qu'entre les bandes de colle vont se former dans le non-tissé des parties en creux, et, après écirement, le non-tissé aura des formes en crêtes et des formes en creux et pourra être étiré.

De préférence, l'épaisseur de la strate d'une pièce ou d'un seul tenant, comportant la ou les zone(s) de renforcement en forme de film(s) et la ou les zone(s) élastique(s) en forme de film(s), au niveau des jonctions entre les zones de renforcement et les zone élastiques est sensiblement constante, notamment constante. En particulier, au niveau des jonctions les deux zones ont chacune sur la région de jonction une épaisseur plus petite qu'à proximité de la jonction, la somme des épaisseurs des deux zones en forme de films au niveau de la jonction étant sensiblement égale à l'épaisseur de l'une ou l'autre des zones en forme de films à proximité de la jonction.

Suivant un autre mode de réalisation, la jonction entre deux zones élastique et de renforcement s'effectue suivant une interface en biseau.

De préférence, la somme des largeurs de la ou des zone(s) élastique(s) en forme de film(s) et de la ou des zone(s) de renforcement en forme de film(s), y compris celle de la zone centrale lorsqu'elle est prévue, est égale sensiblement à la largeur du stratifié, de sorte que les zones de renforcement d'extrémité s'étendent sensiblement exactement jusqu'au bord d'extrémité respectif du stratifié.

Suivant une autre application de grand intérêt également, les largeurs de la ou des zone(s) élastique(s) en forme de film(s) et de la ou des zone(s) de renforcement en forme de film(s), y compris celle de la zone centrale lorsqu'elle est prévue, sont telles que l'une des deux zones de renforcement d'extrémité s'étend sensiblement exactement jusqu'au bord latéral respectif tandis que l'autre des deux zones de renforcement d'extrémité s'étend au delà du bord latéral respectif, de manière à dépasser hors du stratifié.

Suivant encore une autre application de grand intérêt, les largeurs de la ou des zone(s) élastique(s) en forme de film(s) et de la ou des zone(s) de renforcement en forme de film(s), y compris celle de la zone centrale lorsqu'elle est prévue, sont telles que l'une des deux zones de renforcement d'extrémité s'étend sensiblement exactement jusqu'au bord latéral respectif tandis que l'autre des deux zones de renforcement d'extrémité s'étend en deçà dé leur bord latéral respectif, de manière à former une lisière qui n'est constitué que d'un non tissé ou de deux non tissés et d'une couche de colle intermédiaire.

Cependant, pour chacune des zones de renforcement en forme de film, on peut envisager, indépendamment de l'autre, qu'elle s'étende soit exactement jusqu'à son bord latéral respectif, soit au delà de ce bord respectif, soit en deçà de ce bord respectif.

La présente invention se rapporte également à un procédé d'obtention d'une partie à crochets suivant l'invention comme défini à la revendication 10.

De préférence, le film co extrudé constitué du ou des film(s) élastique(s) et du ou des film(s) de renforcement est en un ou plusieurs matériau(x) imperméables, notamment à l'air et/ou aux liquides.

Suivant un autre aspect de l'invention, celle ci vise une couche culotte définissant une partie avant, une partie arrière et une partie d'entrejambe reliant la partie avant et la partie arrière, et deux parties intermédiaires latérales reliant les bords latéraux supérieurs gauche et droit de la partie arrière avec les bords latéraux supérieurs respectivement gauche et droit de la partie avant, chaque partie intermédiaire étant réunie de manière fixe, par exemple par soudure, à un bord latéral supérieur respectif de la partie arrière, et chaque partie intermédiaire comportant un stratifié s'étendant dans une direction transversale entre deux bords latéraux respectivement gauche et droit, comprenant une strate fixée à au moins une couche de non tissé par une couche d'adhésif, notamment interposée entre deux couches de non tissé en étant fixée respectivement par une couche d'adhésif respective à chaque couche de non tissé, la strate étant d'une pièce ou d'un seul tenant et comportant une zone élastique en forme de film, notamment en un matériau élastomérique, caractérisée en ce que :
- la strate d'une pièce ou d'un seul tenant comprend au moins une zone de renforcement d'extrémité en forme de film qui s'étend à partir de la zone élastique en forme de film en allant vers l'un des bords latéraux ;
- aucune zone élastique ne se trouvant entre le dit un des bords latéraux et la zone de renforcement en forme de film; et
- la au moins une zone de renforcement en forme de film étant moins étirable que la dite une zone élastique en forme de film, notamment est moins élastique, et préférablement n'est pas élastique, par exemple est en une matière thermoplastique, et
- la au moins une zone élastique en forme de film est en contact avec une ou les deux couches d'adhésif interposées entre le ou les non tissés et la strate.

Suivant un mode de réalisation avantageux, au moins un élément à crochets, comportant une bande de base et des crochets issus de la bande, est fixé sur le stratifié, un élément à boucles est fixé à la partie avant et l'agencement est tel que la fermeture de la couche autour de la taille du porteur se fait par la coopération entre les crochets de la partie intermédiaire et les boucles de la partie avant.

Suivant un autre mode de réalisation également avantageux, les parties intermédiaires sont fixées à la partie avant de manière non amovible, par exemple par soudure.

La présente invention vise également une couche culotte comme définie à la revendication 11.

A titre d'exemple, on décrit maintenant un mode de réalisation de l'invention en se rapportant aux dessins dans lesquels :
la Figure 1 représente une vue en coupe transversale schématique d'un mode de réalisation d'un stratifié suivant l'invention;
la Figure 2 représente une vue en coupe transversale schématique d'un autre mode de réalisation d'un stratifié suivant l'invention;
la Figure 3 représente une vue en perspective d'une installation de production d'un stratifié suivant l'invention, notamment des stratifiés des figures 1 et 2 ;
la Figure 4 est un schéma représentant un échantillon de matériau élastique ou élastomérique préparé pour en déterminer le SET,
la Figure 5 représente la forme de la courbe d'hystérésis obtenue lors de la détermination du SET d'un matériau, notamment élastique ou élastomérique.
La Figure 6 représente un autre mode de réalisation d'un stratifié suivant l'invention, qui correspond au stratifié de la figure 1 auquel a été ajouté sur le dessus, un élément comportant des crochets.
Les figures 7 à 20 représentent d'autres modes de réalisation possible d'un stratifié suivant l'invention ;
La figure 21 représente une vue en coupe transversale de la strate du mode de réalisation de la figure 1 après avoir été formé par extrusion et avant d'être assemblée à une ou deux couches de non tissé ;
La figure 22 représente une partie de la vue de la figure 21 d'une strate ayant une interface film élastique - film de renforcement réalisée en biseau ;
La figure 23 représente des exemples de courbes donnant l'étirement en fonction de la force pour le test de caractère étirable relatif ;
La figure 24 représente encore un autre mode de réalisation d'un stratifié suivant l'invention ;
La figure 25 représente un mode de réalisation d'une couche culotte suivant l'invention incorporant une oreille ou patte de fermeture à crochets incorporant un stratifié comme décrit aux figures précédentes ;
La figure 26 représente vue en perspective un autre mode de réalisation d'une couche culotte suivant l'invention ; et
La figure 27 représente encore un autre mode de réalisation d'un stratifié suivant l'invention.

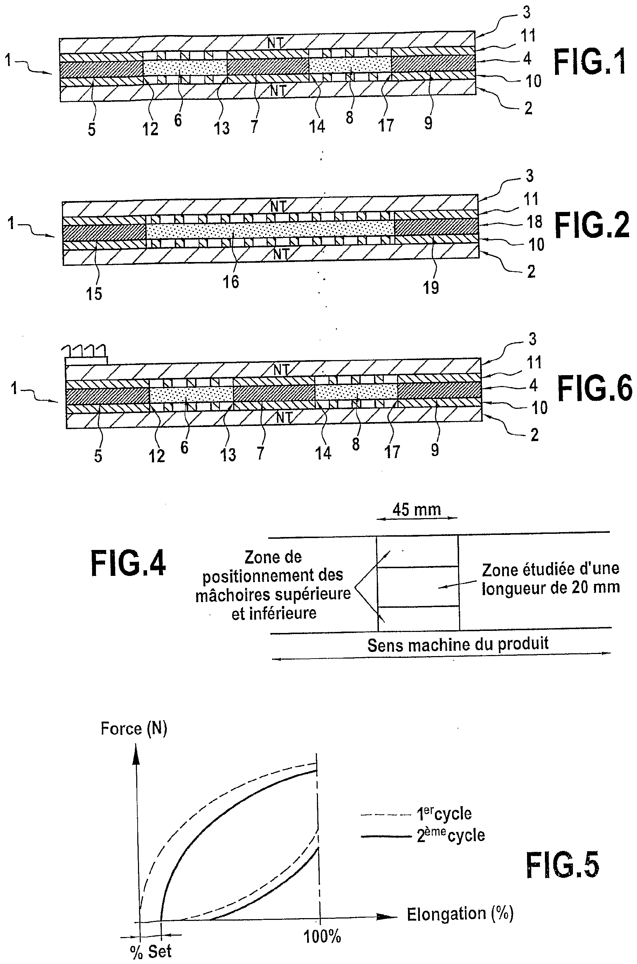

A la Figure 1, il est représenté un stratifié 1 comportant une première couche 2 inférieure de non-tissé et une deuxième couche 3 supérieure de non-tissé. Entre ces deux couches 2 et 3 inférieure et supérieure de non-tissé (à la figure, il est représenté la vue en coupe transversale, sachant que le stratifié dans son ensemble est un élément dont la longueur est perpendiculaire au plan des figures 1 et 2), il est formée une strate 4 intermédiaire. Cette strate 4 intermédiaire est constituée d'un film 5 de renforcement latéral gauche en matière thermoplastique, d'un film 6 élastique gauche en matière élastomérique, d'un film 7 de renforcement central en matière thermoplastique, d'un film 8 élastique droit en matière élastomérique et d'un film de renforcement 9 latéral droite en matière thermoplastique. Des couches 10 et 11 de colles sont interposées entre chaque couche de non tissé et la strate 4 intermédiaire. La largeur totale du stratifié est égale à la largeur de chaque non tissé ainsi qu'à la largeur de chaque couche de colle. La somme des largeurs des trois films de renforcement et des deux films élastiques est égale à la largeur.du stratifié.

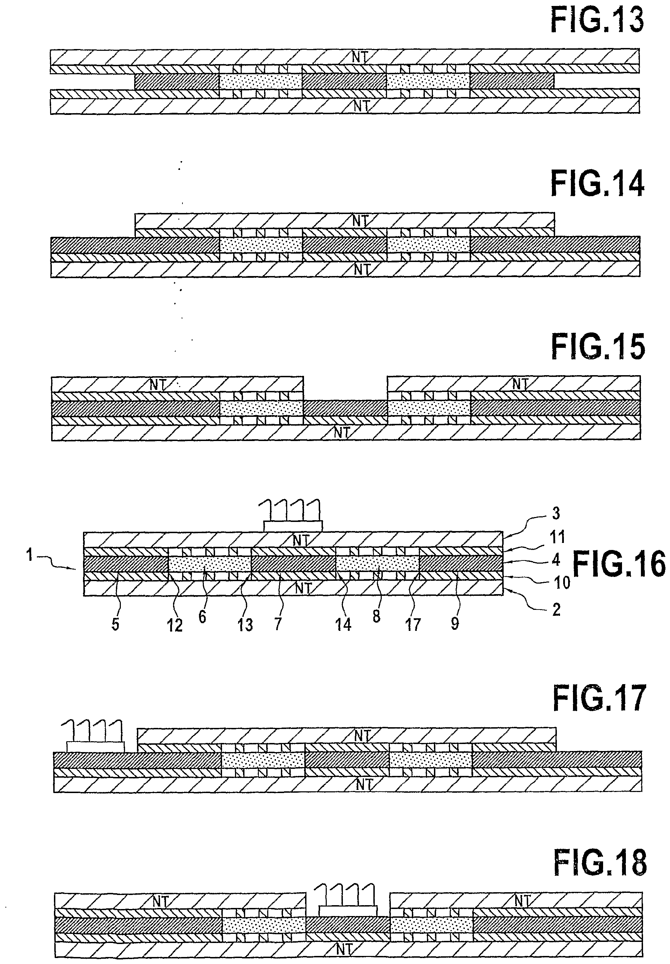

Les deux films de renforcement gauche et droite s'étendent jusqu'au bord respectif gauche et droit du stratifié. On peut également envisager qu'une petite lisière sans bande de renforcement soit formée à chaque bord du stratifié (voir la figure 13) ou qu'au contraire l'un ou les deux films dépassent du bord respectif (voir figures .7, 8, 11 ou 12). Dans tous les cas, l'un des bords des deux films de renforcement d'extrémité est le lieu des points de l'ensemble des films de renforcement d'extrémité qui sont les plus éloignés du ou des films élastiques.

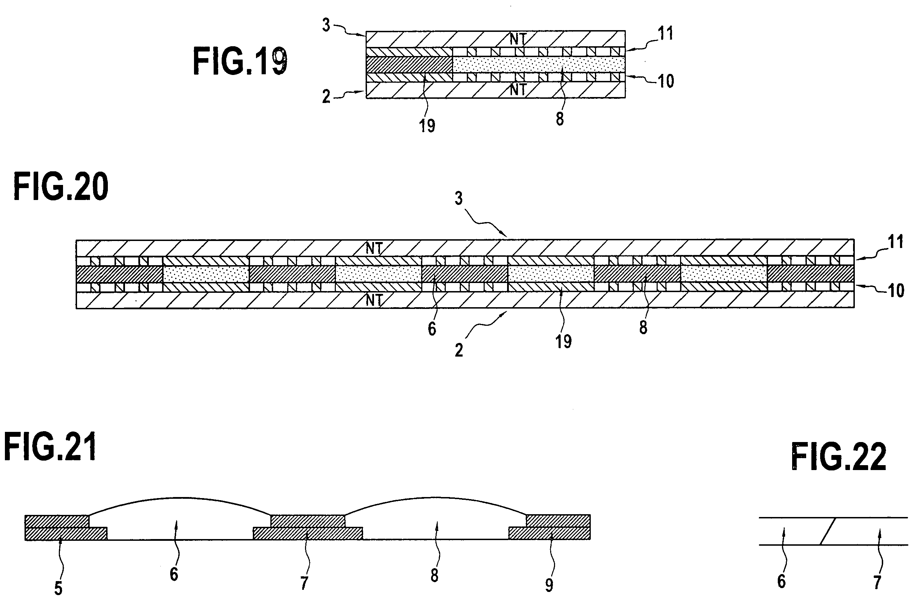

Comme on peut le voir à la figure 21, l'épaisseur de la strate mesurée dans la direction verticale à la figure, c'est-à-dire perpendiculairement à la direction en largeur et à la direction en longueur de défilement est constante ou sensiblement constante sur l'étendue transversale de chaque zone élastique ou de renforcement. Cependant, comme on peut le voir sur cette même figure, l'épaisseur des zones élastiques varie de manière croissante puis décroissante en passant par un maximum entre ses deux jonctions avec les zones de renforcement, mais les épaisseurs du ou des film(s) élastique(s) et de la ou des zone(s) de renforcement sont telles qu'au niveau de leur jonction respective, les surfaces supérieure et/ou inférieure de la strate sont sensiblement sans aspérité ni discontinuité.

Ainsi par exemple, le ou les films élastiques ont un grammage environ 10 à 25% supérieur à celui du ou des films de renforcement, le ou les films élastiques ayant par exemple un grammage de 55 g/m² tandis que le ou les films de renforcement ont un grammage de 45 g/m².

Sur le tronçon de jonction ou liaison, qui s'étend sur une largeur en général comprise entre 3 et 10 mm, notamment 5mm, l'épaisseur de chaque film (de renforcement et élastique) est inférieure à l'épaisseur du film en dehors de la jonction. Ainsi, chaque film de la jonction comporte une partie de plus petite épaisseur qui vient s'assembler en superposition avec la partie de plus petite épaisseur de l'autre film de la jonction. La superposition crée ainsi au niveau de l'interface entre les deux films, comme représenté à la figure 21, une sorte d'échelon. On pourrait également avoir comme forme pour l'interface un biseau comme représenté à la figure 22.

Il peut cependant arriver dans la pratique au niveau des liaisons d'interface entre les deux matières thermoplastique et élastique ou élastomérique, qu'ils se forment de petits renflements liés au fait que la somme des épaisseurs des parties d'extrémité qui se superposent soit légèrement supérieure aux épaisseurs des deux films de part et d'autre de la jonction.

Dans la présente invention, on entend par film ou zone élastique dans une direction donnée un film ou une zone qui reprend sensiblement sa forme initiale après avoir été étiré ou allongé dans la direction donnée, à la température ambiante. De préférence il s'agit d'un film ou d'une zone qui ne conserve qu'une petite déformation résiduelle ou rémanence après déformation et relâchement (permanent set ou SET), à savoir inférieure à 10 %, plus préférablement inférieure à 5 % de la longueur initiale, pour un allongement de 100 % de la longueur initiale de l'échantillon, à température ambiante (T∼ 23°C).

Plus préférablement, le film élastique est capable de subir un allongement à la rupture d'au moins 300 % à température ambiante et, de préférence, un allongement d'au moins 600 %, et plus préférablement d'au moins 1000 %, à température ambiante et pour une vitesse d'étirement de 508 mm/min. Le matériau en lequel est le film élastique peut être soit un élastomère pur, soit des mélanges ayant une phase élastomérique ou une matière qui présente toujours des propriétés sensiblement élastomériques à la température ambiante, comme par exemple des polyoléfines issus de catalyse type métallocène. Il peut également comporter une charge de matière thermoplastique pour entre autre aider à la formation de la strate d'un seul tenant avec les films de renforcements.

Pour mesurer la rémanence, ou SET, d'un échantillon, et donc déterminer s'il s'agit d'un échantillon d'un élément, notamment un film, élastique, on utilise la méthode suivante :
On conditionne l'échantillon dans une atmosphère normale, telle que définie dans la norme ASTDM 5170, température de 23°C ± 2°C et humidité relative de 50% ± 5%.

On utilise comme appareillage un dynamomètre conforme à la norme EN 10002, notamment le Synergie 200H, 1 colonne disponible auprès de la société MTS Systems Corp, U.S.A., conjointement avec un logiciel d'utilisation TESTWORKS 4.04 B.

On prépare l'échantillon en découpant à l'emporte pièce le produit dont on souhaite mesurer l'élasticité en un échantillon de forme rectangulaire (par exemple de 45mm de largeur dans le sens MD (direction de la machine, perpendiculairement au plan de la figure 1) et d'une longueur dans le sens CD (direction transversale, direction horizontale à la figure 1) de 60mm).

On positionne l'échantillon encre les mâchoires de part et d'autre de la zone donc on souhaite tester l'élasticité et de chaque côté, comme représenté schématiquement à la figure 4 ;

Les paramètres sont sélectionnés comme suit :
Distance inter mâchoires : 20mm
Vitesse machine : 254 mm/min
Nombre de cycles : 2
Allongement du produit : 100% à vitesse constante

On étire le produit à 100% par déplacement vertical des mâchoires, après application d'une précharge de l'ordre de 0,05 N, puis on le maintien dans la position pendant 30 secondes (premier cycle), puis on le laisse revenir à la position initiale où on le laisse 60 secondes, puis on l'étire de nouveau à 100%, on le maintien pendant 30 secondes (deuxième cycle) et on revient à la position initiale. On obtient alors la courbe donnant la force d'étirement en N en fonction de l'allongement en %, celle-ci présentant une hystérésis, qui peut alors être caractérisée par le SET (n=2), à savoir le point de la courbe de départ du deuxième cycle qui coupe l'axe des X (élongation en %) à la figure 5.

Plus préférablement, le film élastique est capable de subir un allongement à la rupture d'au moins 300 % à température ambiante et, de préférence, un allongement d'au moins 600 %, et plus préférablement d'au moins 1000 %, à température ambiante et pour une vitesse d'étirement de 508 mm/min. Le matériau en lequel est le film élastique peut être soit un élastomère pur, soit des mélanges ayant une phase élastomérique ou une matière qui présente toujours des propriétés sensiblement élastomériques à la température ambiante, comme par exemple des polyoléfines issus de catalyse type métallocène. Il peut également comporter une charge de matière thermoplastique pour entre autre aider à la formation de la strate d'un seul tenant avec les films de renforcements.

Le matériau du film élastique, notamment élastomérique, peut être un matériau élastique thermo-rétrécissable ou non thermo-rétrécissable. Il peut être formé, en particulier, à partir de polymères, tels que des copolymères de différents types de motif de monomères, par exemple alterné tel que A-B, ou séquencé, par exemple A-A-A-B-B-B, ou statistique, par exemple A-A-B-A-B-B-A-A-A-B-A, dont l'ensemble du réseau obtenu peut avoir différentes structures, soit linéaires de type A-B-A, soit radiales de type A-B, indice n (n>2), soit diblock de type A-B qui sont élastomériques, par exemple les copolymères styrène/isoprène (SI), styrène/isoprène/styrène (SIS), styrène/butadiène/styrène (SBS), styrène-éthylène/butylène-styrène (SEBS), styrène-éthylène/propylène-styrène (SEPS) ou SIBS. Des mélanges de ces élastomères les uns avec les autres ou avec des non élastomères modifiant certaines caractéristiques autres que l'élasticité peuvent également être pris en compte. Par exemple jusqu'à 50 % en poids mais, de préférence, moins de 30 % en poids de polymère peuvent être ajoutés en tant qu'ajout raidisseur, tels que des polyvinyles styrène, des polystyrènes ou des poly α-méthyl-styrène, polyesters époxy, polyoléfines, par exemple des polyéthylènes ou certains acétates d'éthylène/vinyle, de préférence ceux de poids moléculaire élevé.

Le matériau du film élastique peut être, notamment, un styrène-isoprène-styrène, disponible par exemple auprès de la Société Kraton Polymers, sous la dénomination KRATON D(Marque déposée), ou de la société DEXCO POLYMERS LP sous la dénomination VECTOR SBC 4211 (Marque déposée). On peut également utiliser un élastomère thermoplastique de polyuréthane, notamment le PELLATHANE (Marque déposée) 2102-75A de la Société The Dow Chemical Company. On peut utiliser également un styrène-butadiène-styrène, notamment le KRATON D-2122 (Marque déposée) de la société Kraton Polymers , ou le VECTOR SBC 4461 (Marque déposée)de la société Dexco Polymers LP. On peut aussi utiliser un styrène-éthylène/butylène, notamment le KRATON G-2832 (Marque déposée) de la société Kraton Polymers, ou un copolymère séquencé styrène-éthylène-butylène-styrène (SEBS), notamment le KRATON (Marque déposée) G2703. On peut également utiliser un copolymère d'acrylate d'isooctyle et d'acide acrylique suivant des rapports de monomère de 90/10. On peut également utiliser un copolymère séquencé polyamide polyéther PEBAX (Marque déposée) 2533 de la société Arkema.

D'autres matériaux possibles sont des polymères polyoléfines ayant les caractéristiques des élastomères, notamment issus de la catalyse métallocène, tel le VISTAMAXX VM-1120 (Marque déposée), disponible auprès de la société Exxon Mobil Chemical ou des polymères chargés EPDM du type Santoprène.

Dans le cas où de la colle est utilisée comme adhésif pour fixer les films élastiques et les films thermoplastiques aux non tissés et éventuellement les non tissés entre eux, on peut, suivant l'invention, utiliser une colle telle que des colles hot melt non réactive, par exemple la H2511 de Bostick, ou une colle réactive, notamment la AX75E de Bostik, ou des colles PU réactives.

De préférence, ces colles auront une nature chimique similaire ou compatible avec une matière composant le film élastique et/ou le film thermoplastique ou de renforcement décrit ci dessus.

Concernant les non tissés, on peut utiliser des non tissés de type spunbond et/ou meltblown et/ou cardé calandré et/ou spunlace et ayant comme matériau de base un ou des polyoléfines, de préférence du polypropylène, du polyester ou un mélange de ceux ci. En particulier, le non tissé « spunbond » est à base de fibres longues ou filaments, ayant un titre en dTex (masse en gramme divisée par une longueur de 10000 m de fil) compris entre 1 dTex et 6 dTex, par exemple 2,2 dTex. En particulier lorsque le stratifié comporte deux couches de non tissé, les couches peuvent être de types différents et/ou de matériaux différents.

De préférence le ou au moins l'un des non tissés ou les deux non tissés a(ont) un grammage compris entre 5 et 25 g/m2, notamment entre 5 et 15 g/m², plus particulièrement entre 5 et 10 g/m2.

En particulier, lorsque le non tissé est un non tissé qui n'a pas de capacité à s'étirer de soi même et doit être activé, le grammage est compris entre 5 et 20 g/m², notamment est d'environ 15g/m² pour un spunbond ou d'environ 8g/m² pour un meltblown. Lorsque le non tissé n'a pas besoin d'être activé pour que le stratifié est des capacités élastiques, c'est à dire lorsque le non tissé a des capacités à s'étirer de soi même sur un domaine d'allongement allant au moins jusqu'à 50%, de préférence au moins 100%, le grammage est de préférence compris entre 15 et 25 g/m², par exemple est de 22g /m² pour un cardé calandré ou de 25g/m² pour un spunlace. En outre, il est à noter que les deux non tissé, dans le cas où il y en a deux, peuvent être différents l'un de l'autre.

Pour déterminer si un élément, notamment un film, est plus étirable qu'un autre élément, par exemple un autre film, suivant l'invention, on peut utiliser un test d'allongement sous effort jusqu'à la rupture comme suit :
On utilise comme appareillage un dynamomètre conforme à la norme EN 10002, par exemple le même que pour le test d'élasticité ci dessus, à savoir le Synergie 200H, 1 colonne, disponible auprès de la société MTS Systems Corp, USA, conjointement avec un logiciel d'utilisation TESTWORKS 4.04 B. On prépare l'échantillon en forme rectangulaire, par exemple de 45 mm de largeur et d'une longueur de 60 mm. On positionne l'échantillon entre les mâchoires de part et d'autre de la zone dont on souhaite tester l'allongement et de chaque côté, comme représenté schématiquement à la figure 4. Les paramètres sont sélectionnés comme suit :
Distance inter mâchoires : 20 mm
Vitesse machine : 254 mm/min
Allongement du produit : jusqu'à rupture à vitesse constante

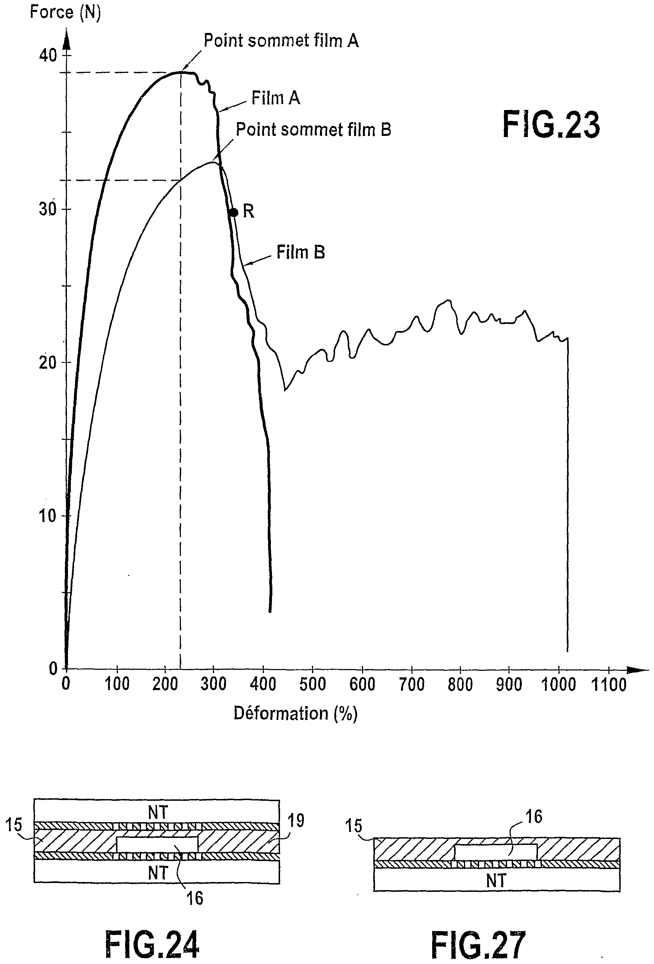

Après avoir appliqué une précharge de 0,05 N, on étire le produit jusqu'à sa rupture par déplacement vertical des mâchoires. On obtient alors pour chaque échantillon la courbe caractéristique donnant la force d'étirement en N en fonction de l'allongement en %, comme représenté à la figure 23. Chaque courbe comporte un point sommet, à partir duquel l'allongement croît à force décroissante. Ce sommet correspond au passage à l'état de ruine de l'élément.

Pour déterminer lequel de deux éléments, notamment de deux films, est le moins étirable, on teste avec la méthode ci dessus les deux éléments, notamment les deux films, de manière à obtenir leur courbe caractéristique respective. Chaque courbe présente un point sommet donné auquel correspond un allongement donné respectif. On choisit alors comme allongement de référence le plus petit des deux allongements donnés des deux courbes ou l'allongement donné dans le cas où les deux, allongements donnés sont identiques et pour cet allongement donné le plus petit on compare les deux forces d'étirement de chaque courbe et l'élément ayant pour cet allongement de référence la force d'étirement la plus grande est l'élément le moins étirable. Ainsi, par exemple à la figure 23, l'allongement de référence est l'allongement correspondant au point sommet du film A, à savoir environ 240% et à cet allongement de référence, la courbe du film A donne une force d'étirement d'environ 40 N et la courbe du film B une force d'étirement d'environ 32,5 N, de sorte que c'est le film A qui est le moins étirable.

Concernant le matériau des bandes de renforcement, on peut choisir une matière ou un mélange de plusieurs matières telles que celles de la famille des thermoplastiques, par exemple le polyéthylène, le polypropylène, le polyester ou toute matière analogue, ainsi que des matières de la famille des élastomères thermoplastiques utilisées pour le film élastique (mais cependant de sorte que le film de renforcement soit dans tous les cas de moindre allongement que le film élastique), ce qui permet d'assurer une bonne compatibilité des matériaux venant former la strate d'un seul tenant. On choisira de préférence un matériau thermoplastique ayant une bonne compatibilité avec le matériau élastique ou élastomérique du film élastique pour que la liaison entre eux le long de leur bord respectif, après refroidissement, soit la plus forte possible. Des exemples précis de matière pour les films de renforcement sont le polyéthylène Riblène MV 10 (marque déposée) de la société Polimeri Europa, ou le MG 9621 de la société Boréalis, ou le LD259 ou LD655 de la société Exxon Mobil Chemical.

Entre la strate 4 intermédiaire constitué des tronçons 5, 6, 7, 8 et 9, il est prévu des couches de colle 10 et 11. Les bandes de colles 10 et 11 sont continues à l'interface entre chaque non-tissé et chaque bande de renforcement et sont constituées de bandes de colle séparées les unes des autres par des zones en forme de bandes sans colle. On pourrait ne pas coller en continu les films de renforcement et les non tissés, par exemple en réalisant à ce niveau là des bandes de colles séparées, ou même un collage par points ou par pulvérisation aléatoire. Les bandes de renforcement et les films élastiques sont déposés entre les deux non-tissés par co-extrusion. Ils sont chacun assemblés par co-extrusion, de sorte que dans l'outillage de co-extrusion, ils viennent en contact le long de leur bords latéraux 12, 13, 14 et 17 respectifs dans un état de matière ramollie et sous pression. Le contact intime des deux bords des deux matières ramollies l'une avec l'autre des bandes élastiques et des bandes de renforcement fait que les deux matières se solidarisent l'une à l'autre dans l'outillage et conserve cette jonction après refroidissement. Ainsi, par exemple, le film élastique peut être à base d'élastomère thermoplastique tandis que les bandes de renforcement peuvent être thermoplastiques, ce qui assure une facilité des fixations mutuelles des deux interfaces sur les bords latéraux.

A la figure 2, il est représenté un autre mode de réalisation dans lequel le stratifié comporte un film 18 constitué d'un film 16 élastique central et deux bandes (15, 19) de renforcement latérales. Dans ce mode de réalisation, il n'y a pas de bande de renforcement centrale et un seul film élastique. Les autres caractéristiques du stratifié de la figure 2 sont identiques à ce qui a été décrit pour le stratifié de la figure 1.

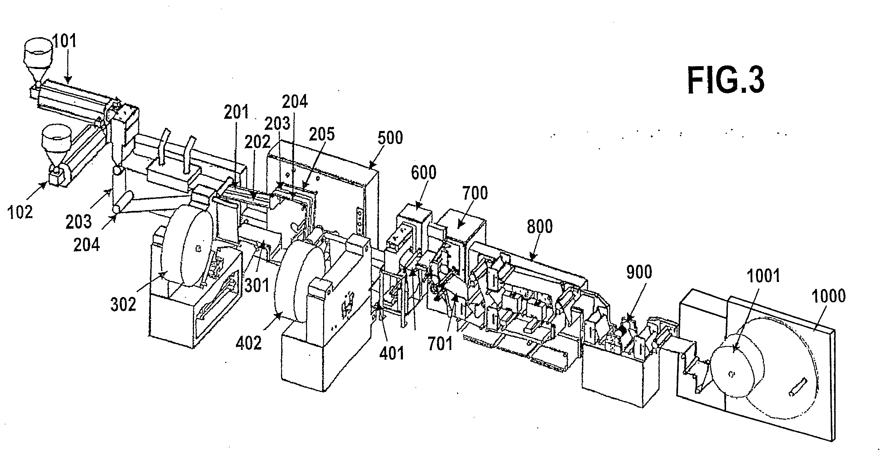

A la figure 3, il est représenté une installation de fabrication d'un stratifié suivant l'invention: l'installation comporte une extrudeuse 101 qui forme par extrusion deux rubans 201, 202 de films élastiques qui par l'intermédiaire de systèmes à courroies 203 et galets 204 sont transportés entre deux rouleaux en même temps que deux couches de non tissé 301 et 401 pour y être fixés par refroidissement de la matière élastomérique entre les non tissés dans une unité de fixation 500. Le non tissé 301 est déroulé à partir d'une bobine 302. Le deuxième non tissé 401 est déroulé d'une bobine 402. De l'extrudeuse 102 sont également formés trois rubans 203, 204, 205 en matière thermoplastique. Les cinq rubans 201 à 205 sont co-extrudés les uns à côté des autres en ayant leur bords contigus, de sorte qu'en sortie d'outillage de co-extrusion et par la suite après refroidissement, ils ne forment plus qu'une unique pièce. Le complexe constitué des non tissés 301 et 401 et des rubans élastiques et thermoplastiques 201 à 205, est à savoir le complexe 601, est ensuite laminé dans une calandre 600, découpé à la bonne largeur dans un système de découpe 700 en largeur comportant des couteaux 701 circulaires, puis passe dans une unité de soudure 800, permettant une soudure des bords longitudinaux. Les non tissés sont enfin activés par défibrillation, dans une unité 900 d'activation.

A la sortie de l'unité 900 d'activation, le stratifié dans son ensemble est enroulé sur un enrouleur 1000 en un rouleau 1001 qui pourra ensuite être amené directement sur les unités de fabrication des couches culottes pour la dépose sur une couche culotte.

De préférence, le ou les films élastiques ou élastomériques ont une épaisseur comprise entre 20 microns et 120 microns." De même les bandes de renforcement ont de préférence la même épaisseur.

Suivant les applications, on peut choisir les dimensions des films de renforcement en matière thermoplastique de sorte que l'une d'entre elle s'étende exactement jusqu'à un des bords gauche ou droit du stratifié, tandis qu'à l'autre bord, soit la bande s'étend également jusqu'au bord droit ou gauche exactement, soit dépasse d'une petite distance à l'extérieur au delà du bord du stratifié, soit au contraire se termine en retrait de ce bord de manière à former une petite lisière, notamment sans film de renforcement.

A la figure 6, on a représenté la stratifié de la figure 1 auquel a été fixé sur le non tissé supérieur, dans la zone en face ou en correspondance avec la bande de renforcement gauche une nappe à crochet en matière thermoplastique, constituée d'une bande en matière thermoplastique de laquelle font saillie des éléments d'accrochage, notamment des crochets. La largeur de cette bande à crochets est inférieure à la largeur de la bande de renforcement. On pourrait également prévoir une telle bande au dessus de l'une ou de toutes les autres bandes de renforcement des modes de réalisations des figures 1 et 2, suivant l'application choisie. La fixation de cette bande ou de ces bandes à crochets peut se faire par adhésif, colle ou soudure notamment à ultrason.

Dans les exemples décrits ci dessus, on a utilisé deux couches de non tissés identiques. On peut également prévoir deux couches en des matières non tissés différentes ou ayant des grammages différents, et on peut également prévoir une seule couche de non tissé au lieu de deux.

Dans les exemples décrits ci dessus, on a extrudé les films de renforcement à partir d'une même extrudeuse 102. On pourrait également prévoir une troisième extrudeuse pour extruder une des zones avec une formulation en matière thermoplastique différente de l'autre zone de renforcement.

Dans les exemples ci dessus, la strate 4 comporte deux films élastiques et trois films de renforcement. On peut également prévoir que cette strate soit composée d'une multiplicité de zones élastiques et de renfort avec un nombre respectif supérieur ou égal à 4.

En outre, on peut prévoir d'ajouter des colorants, sous forme de mélange maître (masterbatch), sous forme liquide ou sous forme de poudre, jusqu'à 7 %, de préférence inférieur à 1%, dans la formulation du ou des films élastiques et/ou du ou des film(s) de renforcement pour en permettre une lisibilité aisée.

De même, la formulation de chaque film est réalisée suivant les règles de l'art usuelle en extrusion et intègre éventuellement des agents de type « processing aids », gonflants, anti UV, antiblock, etc.

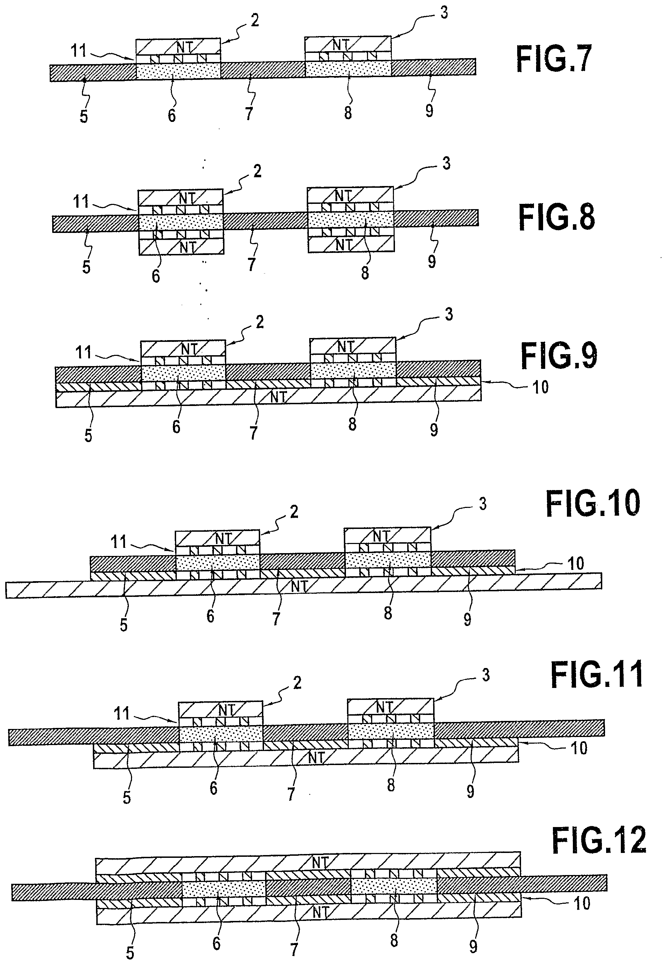

Au figures 7 à 20, sont représentés d'autres modes de réalisation d'un stratifié suivant l'invention, dans lesquels, par rapport aux modes de réalisation des figures 1 et 2, on a fait varier le nombre et/ou les largeurs des couches de non tissé, le nombre de films élastiques, le nombre de films de renforcement, les largeurs des films de renforcement, le positionnement relatif des films de renforcement par rapport aux bords du stratifié, le positionnement d'un élément comportant des crochets. Bien évidemment, il est également contenu dans la présente description les modes de réalisation que l'on obtient en combinant deux ou plusieurs des modes des réalisation des figures 1 , 2, 6 à 18, notamment lorsque l'on prend dans l'un de ces modes de réalisation une ou des caractéristiques que l'on ne retrouve pas dans un autre des modes de'réalisation décrits et que l'on ajoute cette ou ces caractéristiques dudit un de ces modes de réalisation audit un autre des modes de réalisation.
Dans ces figures, les mêmes références numériques qu'au figures 1 et 2 on été utilisées pour décrire des éléments identiques.

A la figure 7, il est décrit un stratifié ne comportant qu'une couche supérieure de non tissé, qui ne s'étend que sur les deux films élastiques 6 et 8. La strate 4 comporte trois films de renforcement 5, 7 et 9 et deux films élastiques 6 et 8.

A la figure 8, il est représenté un mode de réalisation sensiblement identique à celui de la figure 7, à l'exception du fait qu'il comporte également une couche de non tissé inférieure, qui elle aussine s'étend que sur les films élastiques.

A la figure 9, il est représenté encore un autre mode de réalisation qui est sensiblement identique à celui de la figure 7, à l'exception du fait qu'on y a ajouté une couche de non tissé inférieure qui s'étend sur toute la largeur de la strate intermédiaire, exactement d'un bord à l'autre de cette strate.

A la figure 10, il est représenté un mode de réalisation sensiblement identique à celui de la figure 9, à l'exception du fait que le non tissé inférieur s'étend au delà des bords latéraux de la strate intermédiaire.

A la figure 11, il est représenté un mode de réalisation sensiblement identique à celui de la figure 9, à l'exception du fait que le non tissé inférieur s'étend en deçà des bords latéraux de la strate intermédiaire, les deux films de renforcement d'extrémité faisant saillie latéralement au delà des deux non tissés inférieur et supérieur.

A la figure 12, il est représenté un mode de réalisation sensiblement identique à celui de la figure 1, à l'exception du fait que les deux films de renforcement d'extrémité font saillie latéralement au delà des deux non tissés inférieur et supérieur.

A la figure 13, il est représenté un mode de réalisation sensiblement identique à celui de la figure 1, à l'exception du fait que les deux films de renforcement d'extrémité ne s'étendent pas jusqu'aux bords latéraux du stratifié en restant en retrait latéralement par rapport aux deux non tissés.

A la figure 14, il est représenté un mode de réalisation sensiblement identique à celui de la figure 1, à l'exception du fait que la couche supérieure de non tissé ne s'étend pas jusqu'aux bords d'extrémité de la strate intermédiaire, les deux films de renforcements et la couche inférieure de non tissé s'étendant latéralement au delà de la couche supérieure de non tissé.

A la figure 15, il est représenté un mode de réalisation sensiblement identique à celui de la figure 1, à l'exception du fait que la couche supérieure de non tissé ne s'étend pas au dessus du film central, laissant ce dernier à nu, surface à nue qui peut servir à la fixation directe d'un élément comportant des crochets, comme représenté à la figure 18.

A la figure 16, il est représenté un mode de réalisation identique à celui de la figure 6, à l'exception du fait que l'élément comportant des crochets est fixé au non tissé supérieur au niveau du film central et non au niveau d'un des films de renforcement d'extrémité.

A la figure 17, il est représenté le mode de réalisation de la figure 14 auquel a été fixé sur une partie d'extrémité du film de renforcement d'extrémité qui fait saillie au delà de la couche supérieure de non tissé un élément comportant des crochets.

A la figure 18, il est représenté le mode de réalisation de la figure 15 auquel a été fixé sur la partie laissée à nue du film central un élément comportant des crochets.

A la figure 19, il est représenté un stratifié ne comportant qu'un film élastique et qu'un film de renforcement d'extrémité. Le film de renforcement s'étend du film élastique jusqu'au bord latéral (gauche à la figure). Le bord d'extrémité du film de renforcement du côté opposé au dit un film élastique est le lieu des points du film de renforcement d'extrémité les plus éloignés de tout film élastique.

A la figure 20, il est représenté un stratifié dans lequel la strate comporte en alternance cinq films de renforcement et quatre films élastiques.

A la figure 24, il est représenté un mode de réalisation d'un stratifié suivant l'invention, comportant une couche de non tissé supérieure et une couche de non tissé inférieure. La strate intermédiaire comporte en succession, co extrudés ensembles, un film de renforcement, un film élastique et un film de renforcement. Lors de l'extrusion, les deux films de renforcement ont tendance à déborder au dessus du film élastique jusqu'à former une petite pellicule au dessus du film élastique, la pellicule en matériau de renforcement (moins étirable que le matériau élastomérique) reliant les deux films de renfort. La pellicule, par rapport à l'épaisseur de matériau élastique, est suffisamment mince cependant pour que la strate conserve son caractère élastique au niveau du film élastique. La pellicule est en contact direct (avec interposition de colle uniquement) avec le non tissé supérieur. Du côté inférieur, il y a contact direct entre le film élastomérique et le non tissé inférieur(avec interposition de points ou lignes de colle uniquement) et entre les films de renfort et le non tissé inférieur. A la figure 27 , il est représenté une variante du mode de réalisation du stratifié de la figure 24, le stratifié de la figure 27 étant identique au stratifié de la figure 24 à l'exception du fait qu'il ne comporte pas la couche de non tissé supérieure et la couche de colle intermédiaire entre la strate co-extrudée et la couche de non tissé supérieure. Ainsi, la surface supérieure de ce stratifié de la figure 27 est elle constituée uniquement de la matière formant la bande de renfort et la partie en pont au dessus de la couche élastique, avec éventuellement une bande à crochets fixée à cette surface supérieure.

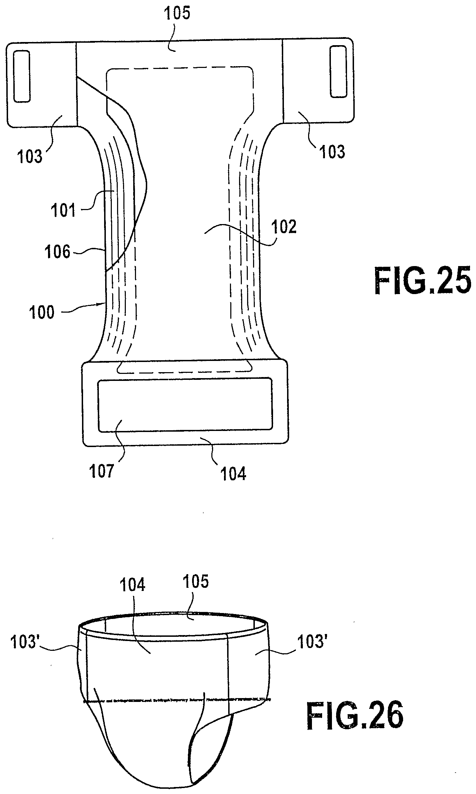

A la figure 25, il est représenté une couche culotte disposée sensiblement à plat et non refermée sur elle même. La couche 100 est constituée d'un châssis formé d'un empilement d'un recouvrement 101 extérieur imperméable et d'une doublure 102 du côté du corps perméable aux liquides corporels. Entre le recouvrement et la doublure, il est disposé un matériau absorbant.

Le châssis forme une partie 104 avant, destinée à se trouver du côté avant du porteur de la couche lorsqu'elle est portée, une partie 105 arrière, destinée à se trouver du côté arrière du porteur de la couche, lorsqu'elle est portée, et une partie 106 d'entrejambe, reliant les parties arrière et avant. Sur la partie avant, sur la face extérieure du recouvrement 101 extérieur, il a été disposé, par exemple par collage, soudure à ultra sons ou analogue une partie 107 à boucles, par exemple sous la forme d'un tricot à boucles, d'un tissé à boucles ou d'un non tissé à boucles.

Des deux bords opposés de la partie arrière du châssis font saillie des parties 103 intermédiaires à crochets constitué d'un des stratifiés décrits précédemment auquel on a fixé des crochets destinés à coopérer avec les boucles issues de la partie avant pour fermer la couche culotte autour de la taille du porteur. En particulier les crochets sont fixés au niveau d'une des bandes de renfort, soit directement dessus, soit avec interposition de la couche de non tissé se trouvant au dessus de la bande de renfort.

Les parties intermédiaires 103 sont réunies à la partie arrière, des deux côté de celles ci, de préférence, comme représenté, sensiblement au niveau supérieur de la partie arrière. Cette réunion peut se faire de manière directe ou indirecte. Par exemple, on peut fixer directement chaque partie intermédiaire à la partie arrière par utilisation d'adhésif, soudure thermique ou à ultra son et analogue. On peut également prévoir une pièce intermédiaire aidant à cette réunion et même faire en sorte que les parties intermédiaire 103 soient réalisées d'une pièce ou d'un seul tenant avec la partie arrière. La fixation de chaque partie intermédiaire aux bords supérieurs latéraux de la partie arrière du châssis de la couche se fait au niveau de la bande de renfort ou d'une des bandes de renfort du stratifié constituant cette partie intermédiaire, soit directement soit avec interposition de la couche de non tissé se trouvant au dessus de cette bande de renfort, la présence de cette bande de renfort facilitant cette fixation.

A la figure 26, il est représenté un autre mode de réalisation d'une couche suivant l'invention. Dans ce mode de réalisation les parties 103' intermédiaires sont réunies à la fois à la partie arrière (comme dans le mode de réalisation de la figure 25) et à la partie 104' avant . d'une manière analogue à la façon dont sont réunies les parties arrières et intermédiaires. Les parties intermédiaires sont également, comme dans le mode de réalisation de la figure 25, constituées d'un des stratifiés décrits précédemment. La seule différence est que dans ce mode de réalisation la partie intermédiaire 103' ne comporte pas de crochets et que la réunion avec la partie avant n'est pas amovible comme c'est le cas dans le mode de réalisation de la figure 25 par la coopération des crochets et des boucles. Ainsi, dans ce mode de réalisation de la figure 26, il n'est pas nécessaire de prévoir une partie à boucles dans la partie avant. La fixation de chaque partie intermédiaire aux bords supérieurs latéraux de la partie arrière et de la partie avant du châssis de la couche se fait au niveau d'une respective des bandes de renfort du stratifié constituant cette partie intermédiaire, soit directement, soit avec interposition de la couche de non tissé se trouvant au dessus de cette bande de renfort, la présence des bandes de renfort facilitant cette fixation.

## Revendications

1. Partie à crochets d'une fermeture auto agrippante à crochets et boucles, notamment pour une couche culotte, comportant un élément à crochets comportant une nappe de laquelle font saillie des crochets et un élément stratifié fixé à l'élément à crochets, le stratifié s'étendant dans une direction transversale entre deux bords latéraux respectivement gauche et droit, comprenant une strate étant d'une pièce ou d'un seul tenant fixée à au moins une couche (2, 3) de non tissé par une couche d'adhésif, notamment interposée entre deux couches de non tissé en étant fixée par une couche d'adhésif respective à chaque couche de non tissé, la strate comportant une zone (6 ; 16) élastique en forme de film, notamment en un matériau élastomérique, **caractérisée en ce que** :
- la strate d'une pièce ou d'un seul tenant comprend au moins une zone (5 ; 15) de renforcement d'extrémité en forme de film qui s'étend à partir de la zone élastique en forme de film en allant vers l'un des bords latéraux ;
- aucune zone élastique ne se trouvant entre le dit un des bords latéraux et la zone de renforcement en forme de film ;
- la au moins une zone (5 ; 15) de renforcement en forme de film étant moins étirable que la dite une zone élastique en forme de film, notamment est moins élastique, et préférablement n'est pas élastique, par exemple est en une matière thermoplastique, et
- la au moins une zone (6 ; 16) élastique en forme de film est en contact avec une ou les deux couches d'adhésif interposées entre le ou les non tissés et la strate.

2. Partie à crochets suivant la revendication 1, **caractérisée en ce que** la strate d'une pièce ou d'un seul tenant comprend deux zones (5, 9 ; 15, 19) de renforcement d'extrémité en forme de films de part et d'autre de la dite une zone élastique en forme de film.

3. Partie à crochets suivant la revendication 1 ou 2, **caractérisé en ce que** la strate est interposée entre deux couches ou nappes de non tissé et fixée respectivement par une couche d'adhésif respective à chaque couche ou nappe de non tissé.

4. Partie à crochets suivant la revendication 1, 2 ou 3, **caractérisé en ce que** la strate d'un seul tenant comporte une deuxième zone (8) élastique en forme de film à distance de ladite une zone élastique en forme de film et, outre les deux zones de renforcement d'extrémité, une zone (7) centrale en forme de film s'étendant entre ladite une zone élastique en forme de film et la deuxième zone élastique en forme de film.

5. Partie à crochets suivant l'une des revendications 1 à 4, **caractérisé en ce que** les épaisseurs du ou des film(s) élastique(s) et de la ou des zone(s) de renforcement sont telles qu'au niveau de leur jonction respective, les surfaces supérieure et/ou inférieure de la strate sont sensiblement sans aspérité ni discontinuité.

6. Partie à crochets suivant la revendication 5, **caractérisée en ce que** au niveau des jonctions les deux films ont chacun une épaisseur plus petite qu'à proximité de la jonction, la somme des épaisseurs des deux films au niveau de la jonction étant sensiblement égale à l'épaisseur de l'un ou l'autre des films à proximité de la jonction.

7. Partie à crochets suivant la revendication 6, **caractérisée en ce que** la jonction entre deux zones élastique et de renforcement s'effectue au niveau de la jonction suivant une interface en forme d'échelon.

8. Partie à crochets suivant l'une des revendications précédentes, **caractérisée en ce que** l'élément à crochets est fixé sur une face extérieure du stratifié, sur une zone qui se trouve au niveau d'une des zones de renforcement d'extrémité en forme de film(s).

9. Partie à crochets suivant l'une des revendications 1 à 8, **caractérisée en ce que** la ou au moins l'une des couches de non tissés a un grammage compris entre 5 et 15 g/m².

10. Procédé d'obtention d'une partie à crochets suivant l'une des revendications 1 à 9, **caractérisé en ce que** les zones en forme de films (5, 7, 9 ; 15, 19) de renforcement et le ou les zones en forme de film(s) (6, 8 ; 16) élastique(s) sont formées par co-extrusion de sorte que les matières formant les zones en forme de films viennent en contact le long de leurs bords latéraux (12, 13, 14, 17) respectifs dans un état de matière ramollie et sous pression dans l'outillage de co-extrusion de sorte que la ou les zone(s) de renforcement en forme de film(s) et la ou les zone(s) élastique(s) en forme de film(s) forment une strate d'une seule pièce ou d'un seul tenant en sortie d'outillage de co-extrusion, et conserve cette unité après refroidissement.

11. Couche culotte comportant une fermeture auto agrippante ayant une partie à boucles et une partie à crochets suivant l'une des revendications 1 à 9, chacune fixée ou solidarisée à la couche culotte de manière à coopérer entre elles pour fermer la couche culotte, notamment au niveau de la ceinture de la couche culotte.

## Patentansprüche

1. Teil mit Häkchen eines Klettverschlusses mit Häkchen und Schlaufen, insbesondere für eine Windel, umfassend ein Element mit Häkchen, umfassend eine Lage, von der Häkchen abstehen, und ein Laminatelement, das an dem Element mit Häkchen befestigt ist, wobei sich das Laminat in einer Querrichtung zwischen zwei lateralen Rändern jeweils links und rechts erstreckt, umfassend ein Laminat, das aus einem Stück oder aus einem einzigen Teil besteht, und das an mindestens einer Schicht (2, 3) aus Vlies durch eine Haftschicht befestigt ist, die insbesondere zwischen zwei Schichten aus Vlies angeordnet ist, indem sie durch eine jeweilige Haftschicht an jeder Schicht aus Vlies befestigt ist, wobei das Laminat eine nachgiebige Zone (6; 16) in der Form eines Films umfasst, insbesondere aus einem elastomeren Material, **dadurch gekennzeichnet, dass**:
- das Laminat aus einem Stück oder aus einem einzigen Teil mindestens eine Endverstärkungszone (5; 15) in der Form eines Films umfasst, die sich von der nachgiebigen Zone in der Form eines Films erstreckt, indem sie zu einem der lateralen Ränder verläuft;
- sich keine nachgiebige Zone zwischen dem genannten einen der lateralen Ränder und der Verstärkungszone in der Form eines Films befindet;
- die mindestens eine Verstärkungszone (5; 15) in der Form eines Films weniger dehnbar ist als die genannte nachgiebige Zone in der Form des Films, insbesondere weniger nachgiebig ist, und vorzugsweise nicht nachgiebig ist, zum Beispiel aus einem thermoplastischen Material besteht, und
- die mindestens eine nachgiebige Zone (6; 16) in der Form eines Films mit einer oder den beiden der Haftschichten in Kontakt steht, die zwischen dem oder den Vliesen und dem Laminat angeordnet sind.

2. Teil mit Häkchen nach Anspruch 1, **dadurch gekennzeichnet, dass** das Laminat aus einem Stück oder aus einem einzigen Teil zwei Endverstärkungszonen (5, 9; 15, 19) in der Form von Filmen auf beiden Seiten der genannten einen nachgiebigen Zone in der Form eines Films umfasst.

3. Teil mit Häkchen nach Anspruch 1 oder 2, **dadurch gekennzeichnet, dass** das Laminat zwischen zwei Schichten oder Lagen aus Vlies angeordnet ist und jeweils von einer jeweiligen Haftschicht an jeder Schicht oder Lage aus Vlies befestigt ist.

4. Teil mit Häkchen nach Anspruch 1, 2 oder 3, **dadurch gekennzeichnet, dass** das Laminat aus einem einzigen Teil eine zweite nachgiebige Zone (8) in der Form eines Films in einer Distanz von der genannten einen nachgiebigen Zone in der Form eines Films umfasst, und sich, abgesehen von den beiden Endverstärkungszonen, eine zentrale Zone (7) in der Form eines Films zwischen der genannten einen nachgiebigen Zone in der Form eines Films und der zweiten nachgiebigen Zone in der Form eines Films erstreckt.

5. Teil mit Häkchen nach einem der Ansprüche 1 bis 4, **dadurch gekennzeichnet, dass** die Dicken des nachgiebigen Films oder der nachgiebigen Filme und der Verstärkungszone oder Verstärkungszonen derart sind, dass auf der Höhe ihrer jeweiligen Verbindung die oberen und/oder unteren Flächen des Laminats im Wesentlichen keine Unebenheiten und Unregelmäßigkeiten aufweisen.

6. Teil mit Häkchen nach Anspruch 5, **dadurch gekennzeichnet, dass** auf der Höhe der Verbindungen die beiden Filme jeweils eine kleinere Dicke aufweisen als in der Nähe der Verbindung, wobei die Summe der Dicken der beiden Filme auf der Höhe der Verbindung im Wesentlichen gleich der Dicke des einen oder des anderen der Filme in der Nähe der Verbindung ist.

7. Teil mit Häkchen nach Anspruch 6, **dadurch gekennzeichnet, dass** die Verbindung zwischen zwei nachgiebigen und Verstärkungszonen auf der Höhe der Verbindung entlang einer Grenzfläche in der Form einer Stufe erfolgt.

8. Teil mit Häkchen nach einem der vorhergehenden Ansprüche, **dadurch gekennzeichnet, dass** das Element mit Häkchen an einer Außenfläche des Laminats befestigt ist, an einer Zone, die sich auf der Höhe einer der Endverstärkungszonen in der Form eines Films (von Filmen) befindet.

9. Teil mit Häkchen nach einem der Ansprüche 1 bis 8, **dadurch gekennzeichnet, dass** die Schicht oder mindestens eine der Schichten aus Vlies eine Grammatur zwischen 5 und 15 g/m² aufweist.

10. Verfahren zum Erhalten eines Teils mit Häkchen nach einem der Ansprüche 1 bis 9, **dadurch gekennzeichnet, dass** die Zonen in der Form von Verstärkungsfilmen (5, 7, 9; 15, 19) und die Zone(n) in der Form eines nachgiebigen Films (von nachgiebigen Filmen) (6, 8; 16) durch Coextrusion derart gebildet werden, dass die Materialien, welche die Zonen in der Form von Filmen bilden, entlang ihrer jeweiligen lateralen Ränder (12, 13, 14, 17) in einem erweichten Materialzustand und unter Druck in dem Coextrusionswerkzeug derart in Kontakt gelangen, dass die Verstärkungszone(n) in der Form eines Films (von Filmen) und die nachgiebige Zone (nachgiebigen Zonen) in der Form eines Films (von Filmen) ein Laminat aus einem einzigen Stück oder aus einem einzigen Teil am Ausgang des Coextrusionswerkzeugs bilden und diese Einheit nach dem Abkühlen beibehalten.

11. Windel, umfassend einen Klettverschluss mit einem Teil mit Schlaufen und einem Teil mit Häkchen nach einem der Ansprüche 1 bis 9, die jeweils an der Windelschicht derart befestigt sind oder damit fest verbunden sind, dass sie miteinander zusammenwirken, um die Windel zu verschließen, insbesondere auf der Höhe des Bunds der Windel.

## Claims

1. Hook part of a self-gripping hook and loop closure, in particular for a nappy, comprising a hook element comprising a sheet from which hooks protrude and a laminated element fixed to the hook element, the laminate extending in a transverse direction between two respective left and right lateral edges, comprising a layer being in one piece or an integral layer fixed to at least one layer (2, 3) of non-woven fabric by a layer of adhesive, in particular interposed between two layers of non-woven fabric by being fixed by one respective layer of adhesive to each layer of non-woven fabric, the layer comprising a resilient zone (6; 16) in the form of film, in particular made of an elastomeric material, **characterised in that**:
- the layer in one piece or integral layer comprises at least one terminal reinforcing zone (5; 15) in the form of film which extends from the resilient zone in the form of film towards one of the lateral edges;
- no resilient zone being located between said one of the lateral edges and the reinforcing zone in the form of film;
- the at least one reinforcing zone (5; 15) in the form of film being less stretchable than said resilient zone in the form of film, in particular being less resilient, and preferably being non-resilient, for example being made of a thermoplastic material, and
- the at least one elastic zone (6; 16) in the form of film is in contact with one or the two adhesive layers interposed between the non-woven fabric(s) and the layer.

2. Hook part according to Claim 1, **characterised in that** the layer in one piece or integral layer comprises two terminal reinforcing zones (5, 9; 15, 19) in the form of films on both sides of said one resilient zone in the form of film.

3. Hook part according to Claim 1 or 2, **characterised in that** the layer is interposed between two layers or sheets of non-woven fabric and fixed respectively by one respective layer of adhesive to each layer or sheet of non-woven fabric.

4. Hook part according to Claim 1, 2 or 3, **characterised in that** the integral layer comprises a second resilient zone (8) in the form of film at a distance from said resilient zone in the form of film and, together with the two terminal reinforcing zones, a central zone (7) in the form of film extending between said one resilient zone in the form of film and the second resilient zone in the form of film.

5. Hook part according to one of Claims 1 to 4, **characterised in that** the thicknesses of the resilient films (s) and of the reinforcing zone(s) are such that in the region of their respective joint, the upper and/or lower surfaces of the layer are substantially without rough edges or discontinuity.

6. Hook part according to Claim 5, **characterised in that** in the region of the joints the two films each have a thickness which is smaller than in the vicinity of the joint, the total thicknesses of the two films in the region of the joint being substantially equal to the thickness of one or other of the films in the vicinity of the joint.

7. Hook part according to Claim 6, **characterised in that** the joint between two resilient and reinforcing zones is formed in the region of the joint along an interface in the form of a bevel.

8. Hook part according to one of the preceding claims, **characterised in that** the hook element is fixed on an external face of the laminate, to a zone which is located in the region of one of the terminal reinforcing zones, in the form of film(s).

9. Hook part according to one of Claims 1 to 8, **characterised in that** the or the at least one of the layers of non-woven fabrics has a grammage ranging between 5 and 15 g/m².

10. Method for obtaining a hook part according to one of Claims 1 to 9, **characterised in that** the zones in the form of reinforcing films (5, 7, 9; 15, 19) and the zone(s) in the from of resilient film(s) (6, 8; 16) are formed by co-extrusion so that in the co-extrusion equipment the materials forming the zones in the form of films come into contact along their respective lateral edges (12, 13, 14, 17) in a softened material state and under pressure so that the reinforcing zone(s) in the form of film(s) and the resilient zone(s) in the form of film(s) form a layer in one piece or integral layer at the outlet of the co-extrusion equipment and maintain this assembly after cooling.

11. Nappy comprising a self-gripping closure having a loop part and a hook part according to one of Claims 1 to 9, each fixed or attached to the nappy so as to cooperate with one another to close the nappy, in particular in the region of the waistband of the nappy.
